# EUROPEAN PATENT SPECIFICATION

(11) **EP 0 256 479 B1**
(45) Date of publication and mention of the grant of the patent: **20.10.1993**
(21) Application number: 87111561.4
(22) Date of filing: 10.08.1987
(51) Int. Cl.: C07C 25/02, C07C 17/12, C07C 17/24, B01J 29/08

(54) **Process for the catalytic transhalogenation of a poly-iodo-benzene**
Verfahren zur katalytischen Transhalogenierung eines Polyiodobenzols
Procédé pour la trans-halogénation catalytique d'un poly-iodo-benzène

(30) Priority: 11.08.1986 IT 2146586
(43) Date of publication of application: 24.02.1988
(73) Proprietor: ECP ENICHEM POLIMERI S.r.l., I-20124 Milano (IT)
(72) Inventor: Paparatto, Giuseppe, I-20092 Cinisello Balsamo Milan (IT)
(74) Representative: Barz, Peter, Dr.

(56) References cited:
- EP-A- 0 181 790
- EP-A- 0 183 579
- GB-A- 2 155 009
- CHEMICAL ABSTRACTS, vol 66, no. 21, 22nd May 1967, page 8819, abstract no. 94444k, Columbus, Ohio, US; G.J.P. AUGUSTIJN et al.: "Equilibration of benzene derivatives. III. Mono- and poly-substituted iodo- and bromobenzenes."
- PATENT ABSTRACTS OF JAPAN, vol. 9, no. 88 (C-276)[1811], 17th April 1985; & JP - A - 59 219 241

## Description

EP-A-181790 and 183579, in the applicant's name, disclose an oxy-iodination of benzene whereby mono-iodo-benzene and small amounts of di-iodo-benzene are formed; while the mono-iodo derivative can be advantageously used on an industrial scale, e.g. for the manufacture of phenol, the di-iodo-benzenes have not yet found a sufficiently wide field of application.

It now has surprisingly been found that particular and suitable operative conditions allow the di-iodo-benzenes to be easily trans-iodinated to mono-iodo-benzene, in the presence of oxygen and of benzene, according to the equation:

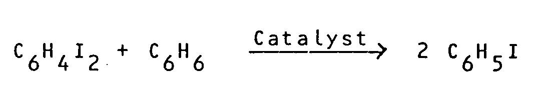

In its broadest aspect the present invention relates to a process for the catalytic trans-iodination of a poly-iodo-benzene, and in particular of a di-iodo-benzene, characterized in that said poly-iodo-benzene is reacted in the gas phase with benzene and oxygen- or an oxygen-containing gas - in the presence of a zeolite of the X type or of the Y type, which is at least partially exchanged with an alkali metal, thallium or a rare earth metal and at any rate is present in a form different from the acidic form, i.e. from the H form, the benzene to poly-iodo-benzene molar ratio ranging from 100:1 to 1:1 and said zeolite being optionally admixed with an inert binder.

The above process can very advantageously be carried out parellel to an oxy-iodination of benzene, disclosed in the above European patent applications, which state that undesired amounts of di-iodo- (and poly-iodo) benzenes are formed; i.e. at the same time, and inside the same reaction zone, higher yields of mono-iodo-benzene can be obtained (as compared to the simple synthesis from C₆H₆ and iodine), because the undesired poly-iodo-benzene by-products can be recycled to the oxy-iodination reaction, thus providing an additional source of iodine.

According to a preferred embodiment of the invention, the space velocity is from 0.1 to 100 kg/hour of (poly-iodo-benzene + benzene) mixture per kg of pure zeolite (any binder excluded) and the di-iodo-benzenes (para-, ortho-, or meta-di-iodo-benzene, or their mixtures) are fed to the reaction as a solution in benzene.

One can use either a zeolite in the mono-alkali-metal form (e.g., in the sodium or potassium form) or a zeolite exchanged with two or more cations. For instance, it is possible to partially exchange the sodium of the sodium form with a different cation, e.g. with the K⁺ cation or with the cation of a rare earth metal. The same catalyst can be also prepared starting from the acidic form of the zeolite by first partially exchanging the proton with one of the desired cations (by using the solution of a water-soluble salt thereof) and subsequently neutralizing all of the residual acidic sites with a diluted NaOH, KOH, RbOH, or CsOH solution; by using this latter technique, a completely exchanged catalyst is obtained, and all of the Bronsted's acidic sites responsible for the decay of the catalytic activity are eliminated.

The trans-iodination can be carried out according to many most different ways which, however, are all within the scope of the present invention. According to a very advantageous embodiment, the reaction temperature is from 250 to 450°C; the benzene/poly-iodo-benzene molar ratio is from 20:1 to 1:1; the poly-iodo-benzene:O₂ molar ratio is from 10:1 to 0.05:1 (preferably from 5:1 to 0.5:1) and the reaction is carried out in a fluidized bed or above a fixed bed of the zeolite catalyst. Further optional operative details are reported hereinafter.

A solution of di-iodo-benzenes in benzene is evaporated and admixed with oxygen or air (oxygen prevents the formation of iodine) and the mixture is fed to a fixed-bed reactor loaded with the catalyst. An inert diluent, e.g. nitrogen, may optionally be used. The products can be recovered by cooling the stream leaving the reactor and resorting to usual treatments. In the case of a distillation, benzene is distilled as the overhead fraction and can be recycled to the reactor. The total pressure is usually not much higher than atmospheric pressure; however, lower or higher pressures can be used. The catalyst maintains its activity for a long time, in particular when the process is carried out, in the gas phase, at 250-450°C; when the catalytic activity decreases under the acceptable level, a regeneration is started; said regeneration may conist of a heat treatment in air, for some hours, at 300-550°C. According to an alternative and very efficient regeneration process, a benzene stream, optionally admixed with air or any other oxygen-containing gas, is passed over the exhausted catalyst at 300-550°C.

The initial activation of the catalyst is also an important step; in general, an activation in air at 450-550°C or the methods disclosed in EP-A-168,978; 169,026; and 169,027 can be used. The invention is described also with the aid of the accompanying figures:
Figure 1 concerns a simple trans-iodination;
Figure 2 deals with parallel oxidative and catalytic mono-iodinations of benzene with iodine.

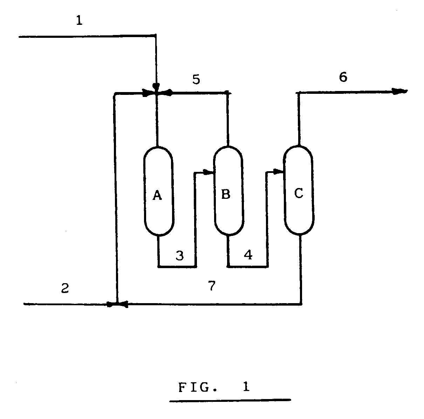

Following the flow diagram of Figure 1, benzene admixed with oxygen (1) and a solution containing one or more di-iodo-benzenes (2) enters, in the gas phase, reactor A loaded with the catalyst; the crude reaction effluent (3) is cooled (inside heat exchangers and heat recovery units, not shown in the Figure) and transferred to separator B, from the bottom of which mono-iodo-benzene and residual di-iodo-benzenes are withdrawn (4), while most of the unreacted benzene and oxygen (5) are recycled to the trans-iodination reaction. Distillation tower C allows the fractionation of the mono- and polyiodinated compounds; mono-iodo-benzene (6) leaves the tower as the overhead fraction and the residual di-iodo-benzenes (7), admixed with a portion of mono-iodo-benzene and with a small amount of benzene, are recycled to the reaction zone.

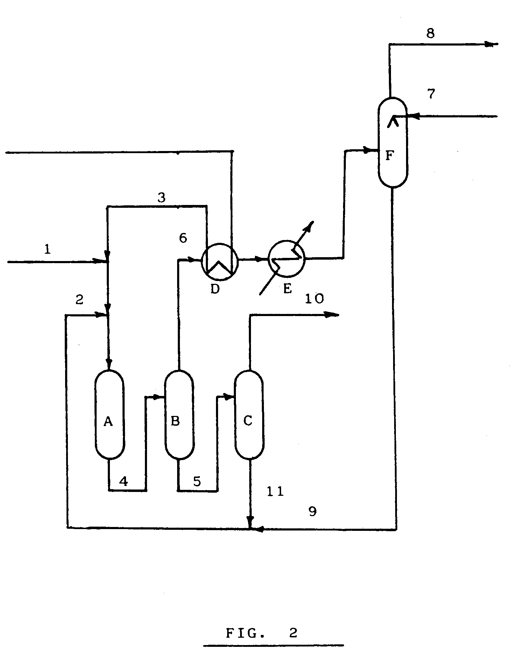

According to Figure 2, a solution containing iodine in an excess with respect to benzene (1) and a benzene solution containing one or more di-iodo-benzenes (2) enter, in the gas phase, reactor A, together with a pre-heated stream of air (or of any other oxidizing gas) (3); the crude effluent from the simultaneous iodination and trans-iodination reactions (4) is cooled (inside facilities not shown in the Figure) and transferred to separator B, from the bottom of which a solution containing mono-iodo-benzene and residual di-iodo-benzenes, together with a small amount of benzene (5) are withdrawn while most of the unreacted benzene, admixed with nitrogen-enriched air (6), is cooled in the recovery unit D and in cooler E before entering the separation and benzene recovery tower F (two towers in series are preferable), where the scrubbing liquid (7) may consist of benzene, mono-iodo-benzene, di-iodo-benzenes or their mixtures. The nitrogen-enriched air (8) can be vented (or transferred to other units) and the excess benzene (9) is recycled. Distillation tower C allows the fractionation of the iodinated compounds; mono-iodo-benzene (10) emanates as the overhead fraction, and the residual di-iodo-benzenes (11) leave the bottom of the tower as tail products and are combined with recycle stream (9). All of the iodine feed is thus completely used, the iodine dispersed in the undesired by-products (poly-iodo-benzenes) being completely recovered (by means of trans-iodination). The following examples illustrate the present invention.

### Example 1 (13 X zeolite)

One gram of 13 X zeolite, sold by Union Carbide, was admixed with 0.3 g of binder (SiO₂) and the whole mixture was activated in air for 2 hours at 540°C; the resulting catalyst was introduced into a quartz microreactor, kept at 400°C and continously fed with a mixture, in the gas phase, of benzene, p-di-iodo-benzene (p-DIB) and air, with a benzene:p-DIB:air molar ratio of 20:1:20. The pressure was slightly higher than 0.1 MPa (760 mmHg) and the weight hourly space velocity (WHSV) was 6 kg/h of (benzene + di-iodo-benzene) mixture per kg of pure zeolite (binder excluded). The reaction continued for 6 hours and the reaction products were collected by condensation; the conversion of p-DIB was 92 % and the selectivity to mono-iodo-benzene (based on converted p-DIB) was 99 %.

### Example 2

Example 1 was repeated using a zeolite NaY (available from Union Carbide); after 6 hours of reaction, the di-iodo-benzene conversion was 80 % and the selectivity to mono-iodo-benzene was higher than 99 %.

### Example 3 (Comparative)

Example 1 was repeated, but the feedstock was a mixture which contained 86 % by weight of benzene, 12 % by weight of iodine and 2 % by weight of para-di-iodo-benzene; after 6 hours of reaction, the iodine conversion was 100 % and the resulting reaction mixture showed the following composition:
- 19.9 % by weight mono-iodo-benzene;
- 1.5 % by weight (para- + ortho-) di-iodo-benzene;
- 78.6 % by weight benzene.

In other terms, under these conditions only 25 % of di-iodo-benzene were trans-iodinated to mono-iodo-benzene.

## Claims

1. A process for the catalytic trans-iodination of a poly-iodo-benzene, characterized in that the poly-iodo-benzene is reacted in the gas phase with benzene and oxygen or an oxygen-containing gas in the presence of a zeolite of the X or the Y type, at least partially exchanged with an alkali metal, thallium or a rare earth metal and present in a form different from the acidic form, the benzene to poly-iodo-benzene molar ratio ranging from 100:1 to 1:1.

2. A process according to claim 1, characterized by recycling of the poly-iodo-benzenes formed during the oxidative catalytic reaction of benzene with iodine and oxygen or an oxygen-containing gas in a reaction zone to said reaction zone.

3. A process according to claim 1 or 2, wherein the temperature is from 250 to 450°C.

4. A process according to any of claims 1 to 3, wherein the benzene:poly-iodo-benzene molar ratio is from 20:1 to 1:1.

5. A process according to any of claims 1 to 4, wherein the poly-iodo-benzene:oxygen molar ratio is from 10:1 to 0.05:1.

6. A process according to any of claims 1 to 5, wherein the space velocity is from 0.1 to 100 kg/h of (poly-iodo-benzene + benzene) mixture per kg of pure zeolite, any binder excluded.

7. A process according to any of claims 1 to 6, wherein the poly-iodo-benzene is fed as a solution in benzene.

8. A process according to any one of claims 1 to 7, wherein the poly-iodo-benzene:O₂ molar ratio is from 5:1 to 0.5:1.

9. A process according to any of claims 1 to 8, wherein the reaction takes place in a fluidized catalyst bed.

10. A process according to any of claims 1 to 8, wherein the reaction takes place above a fixed catalyst bed.

11. A process according to any of claims 1 to 10, wherein the catalyst is regenerated at 300 to 550°C, by means of a benzene stream, optionally admixed with air or any other oxygen-containing gas.

12. A process according to any of claims 1 to 11, wherein the poly-iodo-benzene is a di-iodo-benzene.

## Patentansprüche

1. Verfahren zur katalytischen Transiodierung eines Polyiodbenzols, dadurch gekennzeichnet, daß das Polyiodbenzol in der Gasphase mit Benzol und Sauerstoff oder einem sauerstoffhaltigen Gas in Anwesenheit eines Zeolithen vom X- oder Y-Typ, der wenigstens teilweise mit einem Alkalimetall, Thallium oder einem Seltenerdmetall ausgetauscht ist und in einer von der sauren Form verschiedenen Form vorliegt, umgesetzt wird, wobei das Molverhältnis von Benzol zu Polyiodbenzol im Bereich von 100:1 bis 1:1 liegt.

2. Verfahren nach Anspruch 1, dadurch gekennzeichnet, daß die während der oxidativen katalytischen Reaktion von Benzol mit Iod und Sauerstoff oder einem sauerstoffhaltigen Gas in einer Reaktionszone gebildeten Polyiodbenzole zu dieser Reaktionszone zurückgeführt werden.

3. Verfahren nach Anspruch 1 oder 2, in welchem die Temperatur 250 bis 450°C beträgt.

4. Verfahren nach irgendeinem der Ansprüche 1 bis 3, in welchem das Molverhältnis Benzol:Polyiodbenzol 20:1 bis 1:1 beträgt.

5. Verfahren nach irgendeinem der Ansprüche 1 bis 4, in welchem das Molverhältnis Polyiodbenzol:Sauerstoff 10:1 bis 0,05:1 beträgt.

6. Verfahren nach irgendeinem der Ansprüche 1 bis 5, in welchem die Raumgeschwindigkeit 0,1 bis 100 kg/h (Polyiodbenzol + Benzol)-Mischung pro kg reinem Zeolith beträgt, wobei irgendwelches Bindemittel ausgeschlossen ist.

7. Verfahren nach irgendeinem der Ansprüche 1 bis 6, in welchem das Polyiodbenzol als Lösung in Benzol eingeführt wird.

8. Verfahren nach irgendeinem der Ansprüche 1 bis 7, in welchem das Molverhältnis Polyiodbenzol:O₂ 5:1 bis 0,5:1 beträgt.

9. Verfahren nach irgendeinem der Ansprüche 1 bis 8, in welchem die Reaktion in einem fluidisierten Katalysatorbett stattfindet.

10. Verfahren nach irgendeinem der Ansprüche 1 bis 8, dadurch gekennzeichnet, daß die Reaktion oberhalb eines fixierten Katalysatorbetts stattfindet.

11. Verfahren nach irgendeinem der Ansprüche 1 bis 10, in welchem der Katalysator mit Hilfe eines Benzolstroms, gegebenenfalls mit Luft oder irgendeinem anderen sauerstoffhaltigen Gas vermischt, bei 300 bis 500°C regeneriert wird.

12. Verfahren nach irgendeinem der Ansprüche 1 bis 11, in welchem das Polyiodbenzol ein Diiodbenzol ist.

## Revendications

1. Un procédé de (trans-)iodation catalytique d'un poly-iodo-benzène caractérisé en ce que l'on fait réagir le poly-iodo-benzène en phase gazeuse avec du benzène et de l'oxygène ou un gaz contenant de l'oxygène, en présence d'une zéolite de type x ou de type y, au moins partiellement échangée par un métal alcalin, du thallium ou un métal de terre rare qui est présente sous une forme différente de la forme acide, le rapport molaire benzène/poly-iodo-benzène étant compris entre 100/1 et 1/1.

2. Un procédé selon la revendication 1, caractérisé en ce qu'on recycle les poly-iodo-benzènes formés pendant la réaction catalytique du benzène avec l'iode et l'oxygène ou un gaz contenant l'oxygène dans une zone réactionnelle vers cette zone réactionnelle.

3. Un procédé selon la revendication 1 ou 2, caractérisé en que la température est comprise entre 250 et 450°C.

4. Un procédé selon l'une quelconque des revendications 1 à 3, caractérisé en ce que le rapport molaire benzène/poly-iodo-benzène est compris entre 20/1 et 1/1.

5. Un procédé selon l'une quelconque des revendications 1 à 4, caractérisé en ce que le rapport molaire poly-iodo-benzène/oxygène est compris entre 10/1 et 0,05/1.

6. Un procédé selon l'une quelconque des revendications 1 à 5, caractérisé en ce que la vitesse spatiale est comprise entre 0,1 et 100 kg/heure de mélange (poly-iodo-benzène + benzène) par kilogramme de zéolite pure, tout liant étant exclu.

7. Un procédé selon l'une quelconque des revendications 1 à 6, caractérisé en ce que le poly-iodo-benzène est introduit sous la forme d'une solution benzénique.

8. Un procédé selon l'une quelconque des revendications 1 à 7, caractérisé en ce que le rapport molaire poly-iodo-benzène/O₂ est compris entre 5/1 à 0,5/1.

9. Un procédé selon l'une quelconque des revendications 1 à 8, caractérisé en ce que la réaction est réalisée dans un lit de catalyseur fluidisé.

10. Un procédé selon l'une quelconque des revendications 1 à 8, caractérisé en ce que la réaction est réalisée au dessus d'un lit de catalyseur fixe.

11. Un procédé selon l'une quelconque des revendications 1 à 10, caractérisé en ce que le catalyseur est régénéré une température comprise entre 300 et 550°C au moyen d'un courant de benzène éventuellement mélangé à de l'air ou à un autre gaz quelconque contenant de l'oxygène.

12. Un procédé selon l'une quelconque des revendications 1 à 11, caractérisé en ce que le poly-iodo-benzène est un di-iodo-benzène.
